# EUROPEAN PATENT APPLICATION

(11) **EP 3 449 829 A1**
(43) Date of publication of application: **06.03.2019**
(21) Application number: 16900449.6
(22) Date of filing: 27.04.2016
(51) Int. Cl.: A61B 6/00

(54) **X-RAY APPARATUS**

(71) Applicant: Shimadzu Corporation, Kyoto-shi, Kyoto 604-8511 (JP)
(72) Inventor: MURAKAMI Makoto, Kyoto-shi Kyoto 604-8511 (JP)
(74) Representative: Müller-Boré & Partner Patentanwälte PartG mbB
(86) International application number: PCT/JP2016/063292
(87) International publication number: WO 2017/187579

(57) **Abstract**

An X-ray apparatus is provided with a control circuit. When a duration monitored by a timer in which the device is not operated exceeds a predetermined set time, and a duration monitored by a timer in which a connected state is detected by a transformer exceeds a predetermined set time, the control circuit performs control while disconnecting the connection between a storage cell and a high voltage generation circuit, and performs control while starting the charging of the storage cell via a power socket which is an external power source. This makes it possible, merely by connecting the apparatus main body to the power socket which is an external power source, to disconnect the connection between the storage cell and the high voltage generation circuit and starts the charging of the storage cell on the basis of the results obtained by the timer. As a result, it is possible to automatically charge the storage cell by connecting to the power socket which is an external power source without relying on the actuation and the stoppage of the apparatus.

## Description

### CROSS REFERENCE TO RELATED APPLICATION

This application relates to, and claims priority from, Ser. No.:PCT/JP2016/063292, filed April 27, 2016, the entire contents of which are incorporated herein by reference.

### FIGURE FOR PUBLICATION

Figs. 2.

### BACKGROUND OF THE INVENTION

### Field of the Invention

The present invention relates to an X-ray apparatus comprising an X-ray voltage generation means that generates a voltage for X-ray generation.

### Description of the Related Art

The inventor sets forth a portable X- ray imaging apparatus as an example of the high-voltage generation circuit (voltage generation means for X-ray). A conventional X-ray imaging apparatus that is used for imaging while visiting the room in the hospital, in the patient room on an emergency basis, and in the operation room comprises such as an X-ray tube, an X-ray tube fixing member that fix the X-ray tube, a support arm to support the X-ray tube and the X-ray tube fixing member, a support column to support the support arm, the above described high-voltage generation circuit, and a wheeled platform to carry such components. Nowadays, the portable X-ray imaging apparatus adopting an inverter high-voltage generation circuit having an inverter that transfers the alternating current (AC) to the direct current (DC) is mainly used. The commercial power outlet is not always available for the portable X-ray imaging apparatus, so that an accumulator (rechargeable battery) is embedded inside thereof.

The power source of the accumulator energy accumulation inverter high-voltage generation circuit outputs 100V-300V of DC by connecting the accumulator (rechargeable batteries) in series. The inverter inverts the direct electric current to high-frequency and inputs the high-voltage transformer. Such as the filament power source for the X-ray tube, a rotor rotation power source to rotate the X-ray tube envelope, and the power source for control also generate the respective voltages by an inverter and so forth. The accumulator is recharged by converting the AC power source being supplied from the wall outlet of the commercial electric source to the DC by the converter.

In addition, it is preferred that the accumulator is charged while the portable X-ray imaging apparatus is in a standby time (e.g., referring to Patent Document 1). As Patent Document 1, in the paragraphs [0014] of JP 2015-58297 A1, describes that the portable X-ray diagnosis apparatus 1 is in-place in such as the corridor of each floor in the hospital while standing by and the battery thereof is changed depending on the storage capacity of the battery, the accumulator of the portable X-ray imaging apparatus is recharged while standing by depending on the remaining capacity of the storage battery.

### RELATED PRIOR ART DOCUMENTS

### PATENT DOCUMENT

Patent Document 1 JP 2015-58297 A1

### ASPECTS AND SUMMARY OF THE INVENTION

### Objects to be Solved

Whereas it is a problem that the charging control relative to the accumulator may not be correctly carried out depending on the charging timing of the accumulator. Specifically, when charging the accumulator, inflow of charging electric current or the applied high-voltage on charging, which is a possible cause for a damage of the high-voltage generation circuit or an improper operation, must be avoided. On the other hand, the high-rate discharge of the accumulator is carried out on the X-ray generation and as a result, the rapid voltage drop in the accumulator takes place, so that when the X-ray generation and the charging the accumulator are conducted at the same time, the charge control relative to the accumulator is not correctly achieved.

Now, the high-rate discharge indicates that the large electric current (e.g., 100A) flows in a short period of time (e.g., in millisecond (msec) to second (sec)). In the case of X-ray application, an X-ray is generated by flowing the high-current in a short period of time corresponding to the X-ray irradiation time (msec to sec). Then, the high-current flows from the accumulator in a short period of time, so that the rapid voltage drop in the accumulator takes place.

Therefore, as disclosed in Patent Document 1, JP2015-58297A, the accumulator is charged while the apparatus is in a standby time, i.e., while not-in-use. In such a case, the charging of the accumulator begins following disconnecting the accumulator from the high-voltage generation circuit (X-ray voltage generation means) by manually turning off the power source switch of the apparatus while the apparatus is connecting with the power outlet of the outside power source. Therefore, when charging the accumulator, the accumulator is not charged by just connecting the apparatus with the power outlet, and charging of the accumulator never takes place unless turning off the power switch of the apparatus by disconnecting the accumulator and the high-voltage generation circuit from each other,

Therefore, when the user forgets turning off the switch of the power source of the apparatus despite connecting the apparatus with the power outlet, the charging of the accumulator does not take place, so that the next examination operation may be inoperable due to the power shortage of the accumulator. In addition, whereas the heavy user using the apparatus in full-time wants to arbitrarily charge the accumulator once connected to the power outlet when the apparatus is not being used.

The present invention is completed under consideration of such a circumstance and the purpose of the present invention is to provide an X-ray apparatus that achieves an automatic charging of the accumulator by just connecting with the outside power source regardless of activation-and-suspension of the apparatus.

### [Means for solving the problem]

The present invention comprises the following structure to achieve such objects.

Specifically, an X-ray apparatus according to the aspect of the present invention is the X-ray apparatus having an X-ray voltage generation circuit that generates a voltage for X-ray generation comprises: an accumulator; a timer that monitors a time period; a connection detection circuit that detects a connection between an outside power source and the X-ray apparatus; and a control circuit; wherein the control circuit disconnects the connection between the accumulator and said X-ray voltage generation circuit when a suspension time period, in which the apparatus monitored by the timer is not being operated, is longer than a first preset predetermined time period and the connection detection circuit detects that a connection time period is longer than a second preset predetermined time period, and starts charging the accumulator using an outside power source.

The X-ray apparatus according to the aspect of the present invention comprises an X-ray voltage generator (generation circuit) to generates voltage to generate the X-ray, an accumulator, a timer to monitor the time period, a connection detector to detect the connection between the outside (external) power source and the apparatus per se (main body of the apparatus) and the control unit to achieve the control set forth below. Specifically, a control circuit that disconnects the connection between the accumulator and the X-ray voltage generation circuit when the suspension time period in which the apparatus monitored by the timer is suspending is longer than the first predetermined time period and in addition, when the connection detection circuit detects that the connection time period is over than the second predetermined time period, and initiates to charge the accumulator using the outside power source. In other words, the incident, in which the time when the operation of the apparatus is inactive is over the first predetermined time, means that the apparatus is not in use, and the incident, in which the connection time detected by the connection detector is over the second predetermined time, means that the apparatus per se is being connected to the outside power source over the second predetermined time. Accordingly, just once the apparatus per se is connected to the outside power source, the charging of the accumulator begins following disconnecting the accumulator from the X-ray generation circuit based on the result given by the timer. As a result, the accumulator is automatically charged by just connecting to the outside power source regardless of the activation-and-suspension of the apparatus. In addition, the accumulator is automatically charged by just connecting with the outside power source, so that a possible chance in which the user forgets to charge the accumulator is effectively minimized.

In addition, with respect to the X-ray apparatus according to the aspect of the present invention set forth above, it is preferred that the apparatus comprises a circuit or a component (instrument) that consumes less power than the power consumed by the X-ray voltage generation circuit, and when the apparatus per se is being electrically connected with the outside power source, the power is supplied to such a circuit or an instrumentation from the outside power source, and when the apparatus per se is not being electrically connected with the outside power source, the power is supplied thereto from the accumulator. Now the circuit and instrumentation that consume less power than the power consumed by the X-ray power generation circuit is that the circuit or the instrumentation does not require a power consumption due to a high-current in a short period of time, i.e., not require a high-rate discharge of the accumulator.

Accordingly, when the apparatus per se is being electrically connected with the outside power source, an automatic charging is carried out and even when charging the accumulator and the power supply to such circuit and instrumentation are simultaneously underway, the charging control for the accumulator is correctly achieved without causing the rapid voltage-drop in the accumulator. On the other hand, when the apparatus per se is not electrically connected with the outside power source, the accumulator supplies the power to such circuit and instrumentation. Therefore, regardless of the connection state between the outside power source and the apparatus per se, the power is supplied to such circuit and instrumentation and as a result, the circuit and instrumentation enable a processing.

Such circuit and instrumentation include a below connection-disconnection switching circuit, the above control means (control circuit), a charging circuit relative to charging, a flat panel X-ray detector (FPD), and a personal computer monitor and so forth. The connection-disconnection switching circuit is the switching circuit that connects or disconnects the accumulator with the X-ray power generation circuit and when the apparatus per se is being electrically connected with the outside power source, the power is supplied to connection-disconnection switching circuit from the outside power source, and when the apparatus per se is not being electrically connected to the outside power source, the power is supplied thereto from the accumulator.

Even when the apparatus per se is being electrically connected with the outside power source, the power is supplied to such circuit and instrumentation from the outside power source, and even when the apparatus per se is not being electrically connected with the outside power source and the power is not being supplied thereto from the accumulator. the connection-disconnection switching circuit can be equipped to switch the connection and disconnection between the accumulator and the X-ray voltage generation circuit. One example of such connection-disconnection switching circuit is e.g., a key-switch circuit that is operable through the switching operation from outside.

Three on-and-off modes are available for the key-switching circuit. The first mode is a mode by which once the user turns on the first switch (SW1), each target second switch (SW2) between the two electric circuits and instrumentations is turned on to shunt the two electric circuits and instrumentations to connect electrically to one another. Reversely, the second mode is a mode by which once the user turns on the first switch (SW1), the second switch (SW2) between the two of electric circuits and instrumentations is switched to off to open-and-separate the two circuits and instrumentations, and such a second mode functions as ECO mode to suppress the standby-power (phantom load) due to the connection. Usually, the key-switching circuit is applied to the power switch, so that the key operation, in which the user turns off the first switch (SW1), means that the power switch of the apparatus turns off. Accordingly, once the user turns off the first switch (SW1), the second switches (SW2) between the two targets of electric circuit and instrumentation automatically turn off. Accordingly, once the user turns off the first switch (SW1), the power switch of the apparatus turns off, so that the switching mode, in which the second switches (SW2) between the two targets of electric circuit and instrumentation turn on, is not possible.

When the connection-disconnection switching circuit as such a key-switching circuit, which is externally operable with a switch, is applied to connection-disconnection between the accumulator and the X-ray voltage generation circuit according to the aspect of the present invention, the following features are feasible. With regard to the first mode in which the second switch (SW2) between the accumulator and the X-ray voltage generation circuit turns on to electrically connect: when the first switch (SW1) turns on, the accumulator supplies the power for the X-ray voltage generation circuit and then, the apparatus is usable. With regard to the second mode in which the second switch (SW2) between the accumulator and the X-ray voltage generation circuit turns off to disconnect: when the first switch (SW1) turns on, such a mode is used as ECO mode while the apparatus is in standby. With regard to the third mode in which the second switch (SW2) between the accumulator and the X-ray voltage generation circuit automatically turns off: when the first switch (SW1) off on, the accumulator is solely charged in the third mode.

The connection-disconnection switching circuit as the conventional key-switch circuit, which is externally operable with a switching operation, defers from the control means (e.g., control circuit) as a principal (main) unit and is connected to the accumulator to supply the power to the connection-disconnection switching circuit from the accumulator. According to such a conventional aspect, for example, when the accumulator is charged under the above ECO mode, the high-current from the accumulator that is connected in series does not flow into the X-ray voltage generation circuit separated from the accumulator but flows in the connection-disconnection switching circuit connected with the accumulator. As set forth above, the connection-disconnection switching circuit is the circuit not requiring a high-efficient discharge performance of the accumulator. As a result, the high-frequency current due to the high-current flows into the connection-disconnection switching circuit and may cause a noise.

Then, it is preferred that when the apparatus per se is being electrically connected with the outside power source, the power is supplied to such connection-disconnection switching circuit from such an outside power source, whereas when the apparatus per se is not being electrically connected with the outside power source, the connection-disconnection switching circuit is connected to the accumulator and the power is supplied to such a connection-disconnection switching circuit from the accumulator. In such a case, even when the accumulator is charged under the ECO mode, the apparatus per se is electrically connected with the outside power source on charging the accumulator, so that such outside power source supplies the power to the connection-disconnection switching circuit and it is preventable that the high-current flows into the connection-disconnection switching circuit from the accumulator.

### [Effects of the present invention]

According to the X-ray apparatus of the present invention, when the suspension time period in which the apparatus monitored by the timer is suspending is longer than the first predetermined time period and in addition, when the connection detection circuit detects that the connection time period is over than the second predetermined time period, the connection between the accumulator and the X-ray voltage generation circuit is disconnected and then, the control circuit controls the outside power source so as to begin charging the accumulator, so that such a control circuit cuts the connection between the accumulator and the X-ray voltage generation circuit and begins charging the accumulator based on the result given by the timer just once the apparatus per se is connected to the outside power source. As a result, the accumulator is automatically charged by just connecting to the outside power source regardless of the activation-and-suspension of the apparatus.

The above and other aspects, features and advantages of the present invention will become apparent from the following description read in conjunction with the accompanying drawings, in which like reference numerals designate the same elements.

### BRIEF DESCRIPTION OF THE DRAWINGS

FIG. 1 is a schematic side view illustrating the structure of a mobile X-ray apparatus according to the aspect of the Embodiment.
FIG. 2 is a circuit diagram illustrating an X-ray circuit of the portable X-ray imaging apparatus according to the aspect of the Embodiment.
FIG. 3 is a circuit diagram illustrating a conventional X-ray circuit of the comparative Embodiment with the present Embodiment referring to FIG. 2.
FIG. 4 is a flowchart illustrating that the timer is monitoring the suspension time period while the apparatus is not being operated.
FIG. 5 is a flowchart illustrating that the timer is monitoring the time period of the connection state due to the transformer.
FIG. 6 is a flowchart illustrating the state when each time period being motioned by the timer is over each set-up predetermined time.
FIG. 7 is illustrating on-and-off modes of the key-switching circuit.
FIG. 8 is a circuit diagram illustrating the periphery of the connection detection circuit as the connection detection means according to the aspect of an alternative Embodiment.
FIG. 9 is a circuit diagram illustrating the periphery of the electric current detection circuit as the connection detection means according to the aspect of another alternative Embodiment.

### DETAILED DESCRIPTION OF THE PREFERRED EMBODIMENTS

Reference will now be made in detail to embodiments of the invention. Wherever possible, same or similar reference numerals are used in the drawings and the description to refer to the same or like parts or steps. The drawings are in simplified form and are not to precise scale. The word 'couple' and similar terms do not necessarily denote direct and immediate connections, but also include connections through intermediate elements or devices. For purposes of convenience and clarity only, directional (up/down, etc.) or motional (forward/back, etc.) terms may be used with respect to the drawings. These and similar directional terms should not be construed to limit the scope in any manner. It will also be understood that other embodiments may be utilized without departing from the scope of the present invention, and that the detailed description is not to be taken in a limiting sense, and that elements may be differently positioned, or otherwise noted as in the appended claims without requirements of the written description being required thereto.

Various operations may be described as multiple discrete operations in turn, in a manner that may be helpful in understanding embodiments of the present invention; however, the order of description should not be construed to imply that these operations are order dependent.

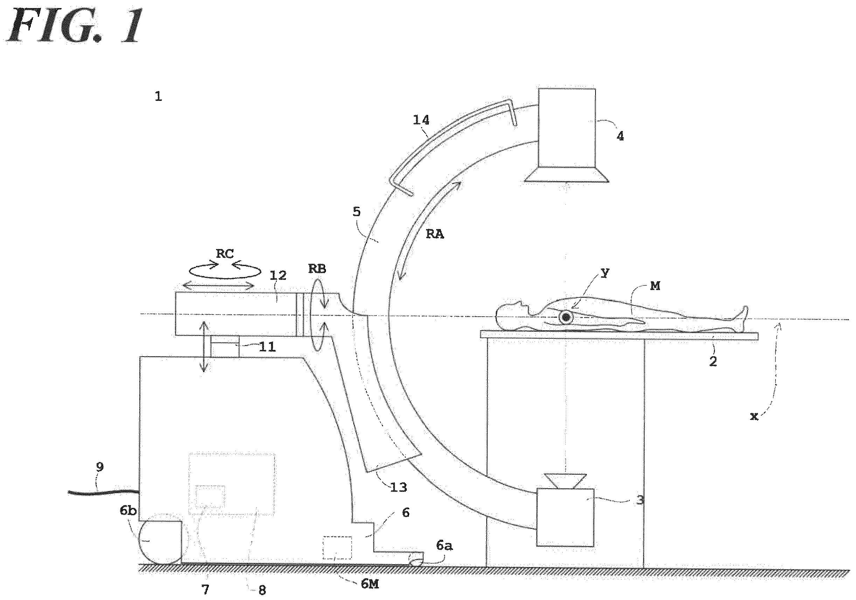

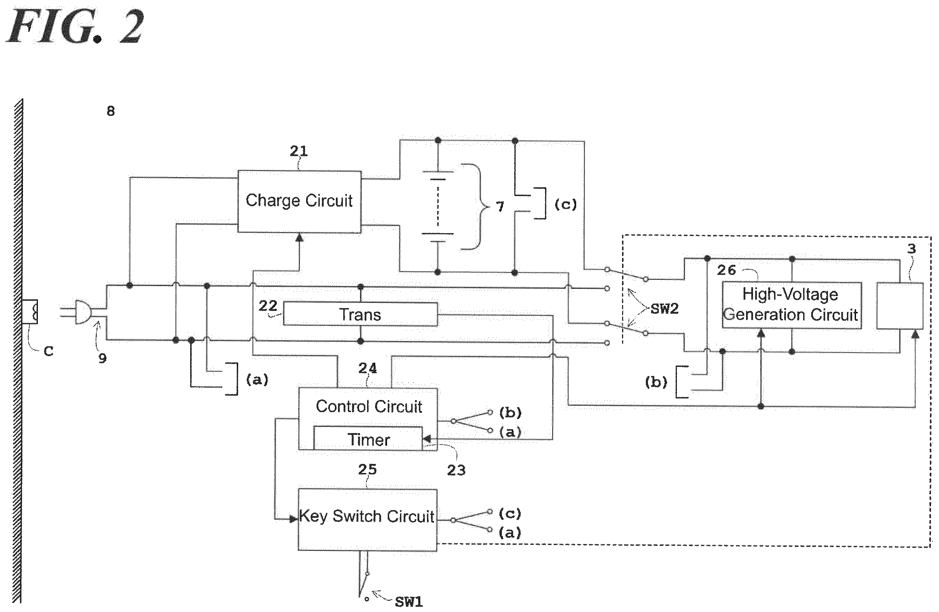

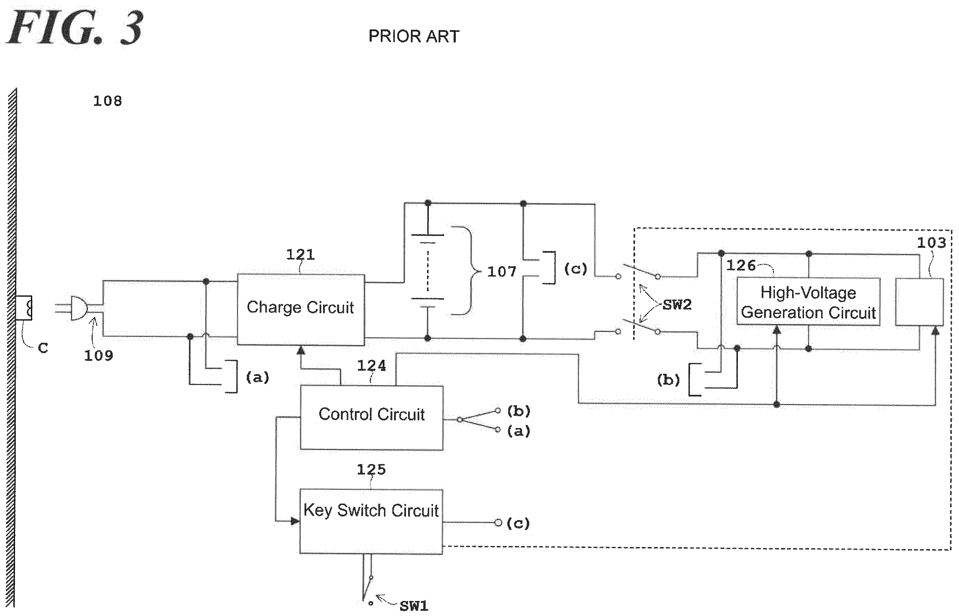

Referring to figures, the inventor sets forth the Embodiment of the present invention. FIG. 1 is a schematic side view illustrating the structure of a mobile X-ray apparatus according to the aspect of the Embodiment, FIG. 2 is a circuit diagram illustrating an X-ray circuit of the portable X-ray imaging apparatus according to the aspect of the Embodiment, and FIG. 3 is a circuit diagram illustrating a conventional X-ray circuit of the comparative Embodiment with the present Embodiment referring to FIG. 2. According to the aspect of the present Embodiment, the inventor sets forth a portable X-ray apparatus as the X-ray apparatus, which is used for imaging while visiting rooms in a hospital, an emergency imaging in the patient room, and an imaging in the operating room (including fluoroscopy on a non-invasive examination) as examples. Here, the non-invasive examination is an examination pursuant to a surgery.

Referring to FIG. 1, the portable X-ray imaging apparatus 1, according to the aspect of the present Embodiment, comprises the X-ray tube 3 (also referring to FIG. 2) and the X-ray detector 4 that move independently from the table 2 (e.g., a surgical table, a bed and a table) on which a subject M is loaded. The X-ray tube 3 irradiates X-ray toward the subject M and the X-ray detector 4 detects the X-ray that transmits through the subject M. Referring to FIG. 1, the X-ray detector is an image intensifier (II), but the present invention is not particularly limited thereto as long as the X-ray detector is ordinarily used, e.g,, a flat panel X-ray detector (FPD). Particularly, when performing a digital imaging, the FPD is more useful. The portable X-ray apparatus 1 corresponds to the X-ray apparatus of the present invention.

Other than the above, the portable X-ray apparatus 1 comprises a C-arm 5 of which one end holds the X-ray tube 3 and the other end holds the X-ray detector 4, a movable wheeled platform 6 movable horizontally relative to the floor surface, a monitor (not shown in FIG.) that displays the X-ray image obtained by the X-ray detector 4 and a hand switch (not shown in FIG.) gripped by a user such as an operator. The movable wheeled platform 6 embeds an accumulator 7 (also referring to FIG. 2) and an X-ray circuit 8 (also referring to FIG. 2) and in addition, a power cable 9 (also referring to FIG. 2) equipped with a plug that is inserted into the power outlet C (also referring to FIG. 2) of the outside power source.

The C-arm 5 is held by the movable wheeled platform 6 and movable relative to the movable wheeled platform 6. The C-arm 5 is curved in the rotation axis x-direction. According to the aspect of the present Embodiment, the C-arm 5 rotates around the center of y-axis (direction indicated by the arrow RA) orthogonal to the rotation center x-axis along the C-arm 5 per se, so that the X-ray tube 3 and the X-ray detector 4 held by the C-arm 5 are rotatable in the same direction. Further, the C-arm 5 rotates around the center of the rotation center x-axis (direction indicated by the arrow RB), so that the X-ray tube 3 and the X-ray detector 4 are rotatable in the same direction, and the C-arm 5 rotates around the axis center of the vertical axis (direction indicated by the arrow RC), so that the X-ray tube 3 and the X-ray detector 4 are rotatable in the same direction.

Specifically, referring to FIG. 1, the C-arm 5 is held by the movable wheeled platform 6 through the support column 11, the horizontal support member 12, and the arm support member 13. The support column 11 is liftable up-and-down along the vertical axis and rotatable and enables lifting the X-ray tube 3 and the X-ray detector 4 together with the C-arm 5 supported by the support column 11. The horizontal support member 12 is movable back-and-forth in the horizontal direction parallel to the rotation center x-axis direction and enables moving the X-ray tube 3 and the X-ray detector 4 together with the C-arm 5 supported by the horizontal support member 12 back-and-forth.

Further, the arm holding member 13 relative to the horizontal support member 12 is held to be rotatable around the axis of the rotation center x-axis, so that the X-ray tube 3 and the X-ray detector 4 are rotatable around the axis center of the x-axis (direction indicated by the arrow RA) together with the whole C-arm 5 supported by the arm holding member 13. The C-arm 5 is held rotatably around the axis center of y-axis orthogonal to the rotation center x-axis relative to the arm support member 13, so that the X-ray tube 3 and the X-ray detector 4 are rotatable around the axis center of the y-axis (direction indicated by the arrow RB) together with the whole C-arm 5. The horizontal holding member 12 relative to the support column 11 is held around the axis center of the vertical axis, so that the X-ray tube 3 and the X-ray detector 4 are rotatable around the axis center of the vertical axis (direction indicated by the arrow RC) together with the whole C-arm 5 that is held by the horizontal holding member 12 and the arm holding member 13.

In addition, the C-arm 5 is equipped with a grip (handle) 14 for manual operation, so that the operator such as an user grips the handle 14 for the manual operation, manually rotates the X-ray tube 3 and the X-ray detector 4 together with the whole C-arm 5 around the axis center of the y-axis (in the direction indicated by the arrow RA), manually rotates the X-ray tube 3 and the X-ray detector 4 together with the whole C-arm 5 around the axis center of the rotation center x-axis (in the direction indicated by the arrow RB), and manually rotates the X-ray tube 3 and the X-ray detector 4 together with the whole C-arm 5 around the axis center of the vertical axis (in the direction indicated by the arrow RC) In addition, the support column 11 is manually moved up-and-down along the vertical axis, the X-ray tube 3 and the X-ray detector 4 are manually moved up-and-down together with the whole C-arm 5 held by the support column 11, the horizontal support member 12 are manually moved back-and-forth in the horizontal direction and the X-ray tube 3 and the X-ray detector 4 are moved back-and-forth together with the whole C-arm 5 held by the horizontal holding member 12.

In addition, the respective weights of the X-ray tube 3, the X-ray detector 4, the movable wheeled platform 6, the support column 11, the horizontal supporting member 12 and the arm holding member 13 are balanced each other based on the structural design, so that even when any component moves to anywhere, the axis never tilts due to shifting of weights. Accordingly, the operator such as the user can easily and manually move the respective components.

The movable wheeled platform 6 has the front wheel 6a and the rear wheel 6b at the bottom thereof. The motor 6M drives the front wheel 6a and the operator such as the user push-and-pulls the movable wheeled platform 6 to rotate the rear-wheel 6b, so that the X-ray tube 3 and the X-ray detector 4 are freely movable in the horizontal direction relative to the floor surface together with the whole C-arm 5. In such a way, with regard to the movable wheeled platform 6 according to the aspect of the present Embodiment, the motor 6M facilitates (assists) manual movements. Needless to say, when the total weight of the portable X-ray apparatus 1 including the X-ray detector 4, the C-arm 5, the movable wheeled platform 6, the support column 11, the horizontal support element 12 and the arm support member 13 is light, the motor is not mandatory to facilitate (assist) such a manual movement. In addition, the motor, a driving axis and a pinion (any is not shown in FIG.) can be equipped to assist the manual movement of the X-ray tube 3, the X-ray detector 4 and the C-arm 5.

In such a way, the C-arm 5 and the movable wheeled platform 6 are manually movable and as a result, both the X-ray tube 3 and X-ray detector 4 are movable relative to the subject M. In addition, a fluoroscopy or an imaging is performed based on the X-ray detected by the X-ray detector 4. When performing the imaging, the X-ray detector 4 detects the X-ray of the normal dose irradiated from the X-ray tube and transmits through the subject M, outputs the X-ray image following a variety of processings including lag correction and gain correction relative to the obtained X-ray detection signal, write on a memory medium (not shown in FIG.) formed from such as RAM (random-access-memory) and store therein, and then later, reads out such an X-ray image from the memory medium arbitrarily according to necessity and then displays such an X-ray image on the display (not shown in FIG.) or prints out such an X-ray image or develops on the film (not shown in FIG.) and so forth. Whereas when performing the fluoroscopy, the X-ray detector 4 detects the X-ray of the less dose than the imaging continuously irradiated from the X-ray tube 3 and transmits through the subject M, outputs the respective X-ray images sequentially and continuously to display on the monitor following a variety of processings including lag correction and gain correction relative to the obtained each X-ray detection signal. In such a way, the X-ray images are directly output to monitor and displayed thereon without once stored in a memory, so that the respective X-ray images obtained by the fluoroscopy are displayed in real-time as a video. In addition, the respective X-ray images obtained by the fluoroscopy can be stored in the memory medium.

Referring to FIG. 2, the X-ray circuit 8 (also referring to FIG. 1) comprises a charging circuit 21 with a converter, a control circuit having the transformer 22 and the timer 23 and the high-voltage generation circuit 26 with a key-switching circuit 25 and an inverter in addition to the accumulator 7 (also referring to FIG. 1) set forth above, and further comprises the X-ray tube 3 (also referring to FIG. 1) set forth above. The transformer 22 corresponds to the connection detection means of the present invention, the control circuit 24 corresponds to the control means of the present invention, the key-switching circuit 25 corresponds to the connection-disconnection switching circuit and the high-voltage generation circuit 26 corresponds to the X-ray voltage generation means of the present invention.

The power cable 9 (also referring to FIG. 1) electrically connects with the charging circuit 21 and the transformer 22, the key-switching circuit 25 connects with the power cable 9 by switching (referring (a) in FIG. 2) via a plurality of circuits (not shown in FIG.) and the control circuit 24 connects with the power cable 9 by switching (referring (a) in FIG. 2). A plurality of circuits, as set forth above, are involved in (a) denoted in FIG. 2, but (a) is not related to the feature of the present Embodiment, so that the detail and the FIG are not provided here.

In addition, the electric outlet C and the high-voltage generation circuit 26 are connected to or disconnected from each other through the power cable 9 by the second switch SW2 in the key-switching circuit 25. In addition, the accumulator 7 and the high-voltage generation circuit 26 are connected to or disconnected from each other by switching using the second switch SW2. When the second switch SW2 electricity connects the accumulator 7 to the high-voltage generation circuit 26, the control circuit 24 is switched-connected with the accumulator 7 in the downstream of the second switch SW2 (referring (b) in FIG. 2) through a plurality of circuits (not shown in FIG.). A plurality of circuits, as set forth above, are involved in (b) denoted in FIG. 2 as well as (a) denoted in FIG. 2, but (b) is not related to the feature of the present Embodiment, so that the detail and the FIG are not provided here.

The charging circuit 21 electrically connects with the accumulator 7 in series and the key-switching circuit 25 switch-connects with the accumulator 7 (referring (c) in FIG. 2) via a plurality of circuits (not shown in FIG.). A plurality of circuits, as set forth above, is involved in (c) denoted in FIG. 2 as well as the (a) in FIG. 2 and the (b) in FIG. 2, but (c) is not related to the feature of the present Embodiment, so that the detail and the FIG are not provided here. In addition, the accumulator 7 and the high-voltage generation circuit 26 are connected with or disconnected from each other by the second switch SW2 in the key-switching circuit 25.

The secondary circuit of the transformer 22 electrically connects with the timer 23. The control circuit 24 switch-connects with any one of the power outlet C (referring (a) in FIG. 2) and the accumulator 7 (referring (b) in FIG. 2) via the power cable 9. The key-switch circuit 25 switch-connects with any one of the power outlet C (referring (a) in FIG. 2) and the accumulator 7 (referring (c) in FIG. 2) via the power cable 9. The control circuit 24 electrically connects with the charging circuit 21, the key-switching circuit 25, the high-voltage generation circuit 26 and the X-ray tube 3. Even not shown in FIG. 2, the control circuit 24 also electrically connects with the X-ray detector 4 (referring to FIG. 1) and the motor 6M (referring to FIG. 1) The control circuit 24 comprises a circuit having such as a central processing unit (CPU) and so forth.

The high-voltage generation circuit 26 connects with the X-ray tube 3. The high-voltage generation circuit 26 includes the inverter, and in fact, the transformer (not shown in FIG.) is installed between the high-voltage generation circuit 26 and the X-ray tube 3, and the high-voltage generation circuit 26 and the X-ray tube 3 magnetically connect with each other (i.e., mutual induction).

FIG. 3 is a circuit diagram illustrating a conventional X-ray circuit as the comparative Embodiment with the present Embodiment. Referring to FIG. 3, the conventional X-ray circuit 108 comprises the accumulator 107, the charging circuit 121 with the converter, the control circuit 124, the key-switching circuit 125, the high-voltage generation circuit 126 with the inverter and in addition, the X-ray tube 103. The points different from the present Embodiment are that the conventional circuit does not have the transformer 22 (referring to FIG. 2) of the present Embodiment, and that the key-switching circuit 125 connects only with the accumulator 107.

In addition, whereas, according to the aspect of the present Embodiment, the second switch SW2 connects and disconnects the accumulator 7 and the high-voltage generation circuit 26 by switching, according to the aspect of the conventional X-ray circuit, when the second switch SW2 is off, the connection between the accumulator 107 and the high-voltage generation circuit 126 is cut off, and when the second switch SW2 is on, the accumulator 107 and the high-voltage generation circuit 126 are electrically connected to each other. Specifically, whereas the second switch SW2 according to the aspect of the present Embodiment is a double-throw switch (i.e., two separate circuits are always switched together), the conventional second switch SW2 is a single-throw switch (i.e., a single circuit is switched on and off.) In addition, the double-throw switch is usable as the single-throw switch. In addition, the conventional timer has a control circuit 124 (not shown in FIG. 3), but the conventional timer is inoperable to monitor the time while the apparatus is not running as the present Embodiment and the connection time period by such as the connection detection means (circuit) represented by the transformer 22 according to the aspect of the present Embodiment.

According to the aspect of the present Embodiment, the plug of the power cable 9 is inserted into the power outlet C to connect the apparatus per se with the power outlet C. The charging circuit 21 having the converter (rectifier), as set forth above, controls converting AC, supplied from AC source through the power outlet C, to DC and charging the accumulator 7 when the apparatus per se electrically connects with the power outlet C.

When the apparatus per se electrically connects with the power outlet C, the control circuit 24 connects with the power outlet C (referring (a) in FIG. 2) through the power cable 9 by switching and the power is supplied to the control circuit 24 form the power outlet C. As well as, when the apparatus per se electrically does not connects with the power outlet C, the key-switch circuit 25 connects with the power outlet C (referring (a) in FIG. 2) through the power cable 9 by switching and the power is supplied to the key-switch circuit 25 form the power outlet C.

On the other hand, when the apparatus per se is not connected with the power outlet C, the second switch SW2 of the key-switching circuit 25 is switched, so that the accumulator 7 and the high-voltage generation circuit 26 connect with each other (referring (b) in FIG. 2), and as a result, the power is supplied to the control circuit 24 from the accumulator 7. As well as, when the apparatus per se electrically does not connects with the power outlet C, the second switch SW2 is switched, so that the key-switch circuit 25 connects with the accumulator 7 (referring (c) in FIG. 2), and as result, the power is supplied to the key-switch circuit 25 form the accumulator 7.

The transformer 22 detects the potential difference of the power cable 9 and sends the detection result (the secondary electric current or the secondary voltage) to the timer 23 from the secondary circuit of the transformer 22. When the apparatus per se electrically connects with the power outlet C, the transformer 22 detects the potential difference between the voltage wire of the power cable 9 and the grounding wire, so that it is detectable that the apparatus per se electrically connects with the power outlet C. Reversely, when the apparatus per se does not electrically connect with the power outlet C, no potential difference between the voltage wire of the power cable 9 and the grounding wire occurs, so that no electric current and no voltage occurs in the secondary circuit of the transformer 22 and as a result, it is detectable that the apparatus per se electrically does not connects with the power outlet C.

When the apparatus is not under the operation (e.g., standby state), a signal relative to the control directive is not input into the control circuit 24. If t₁ is the time while the signal relative to the control directive is not being input into the control circuit 24 and t₂ is the time while the power outlet C and the apparatus per se are being connected with each other, which is detected by the transformer 22, the timer 23 monitors each time ti, t₂. When each time t₁, t₂ becomes over the preset predetermined time, the control circuit 24 disconnects the accumulator 7 and the high-voltage generation circuit 26 using the second switch SW2 in the switching circuit 25 via the key-switching circuit 25 and starts charging the accumulator 7 through the power outlet C.

Specifically, the time while the apparatus monitored by the timer 23 is not running (i.e., t₁ that is the time while the signal relative to the control directive is not being input into the control circuit 24) becomes longer than the preset predetermined time and in addition, the time t₂ under the connection state due to the transformer 22 monitored by the timer 23 becomes longer than the preset predetermined time, the control circuit 24 cuts off the connection between the accumulator 7 and the high-voltage generation circuit 26 and starts charging the accumulator 7 through the power outlet C. In addition, the user can set up such each predetermined time.

Reversely, when the time t₁ while the apparatus monitored by the timer 23 is shorter than the preset predetermined time, or the time t₂ under the connection state due to the transformer 22 monitored by the timer 23 is shorter than the preset predetermined time, at least, charging the accumulator 7 through the power outlet C does not start.

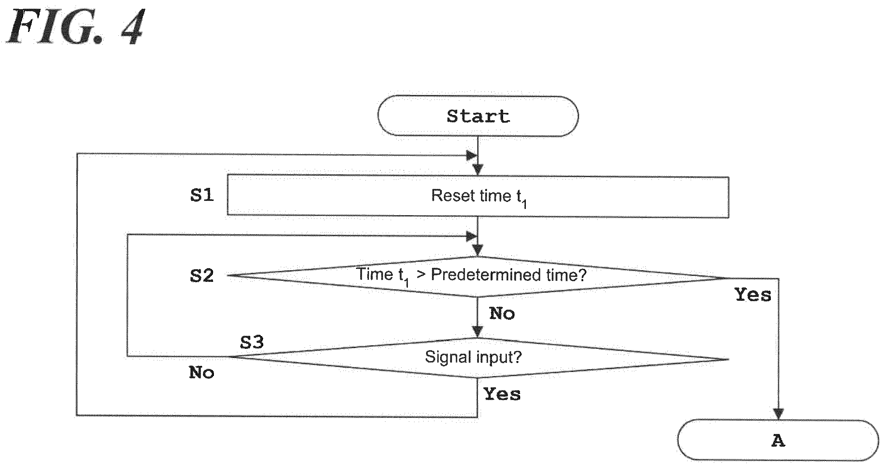

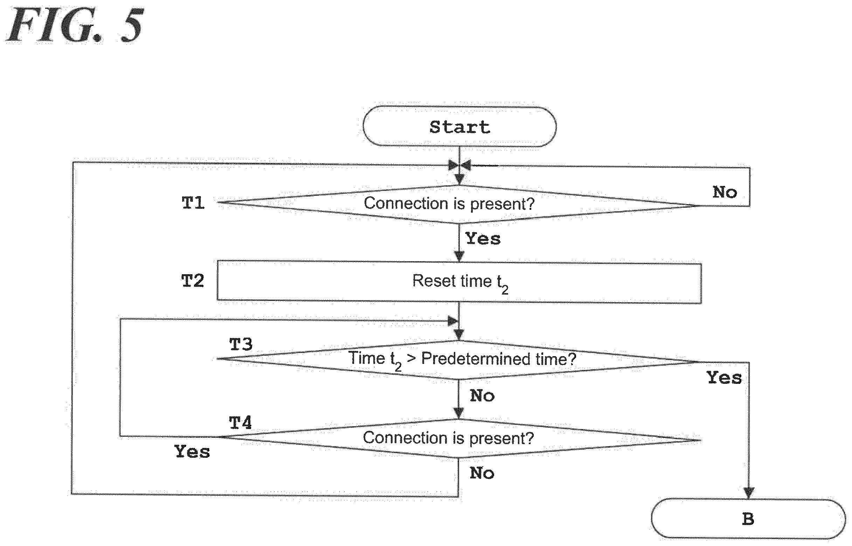

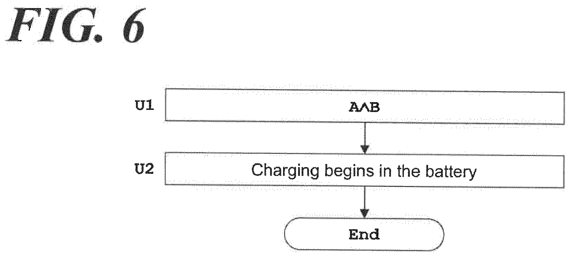

Next, referring to FIG. 4-FIG. 6 in addition to FIG. 2, the inventor set forth each control. FIG. 4 is a flowchart illustrating that the timer is monitoring the suspension time period while the apparatus is not being operated, FIG. 5 is a flowchart illustrating that the timer is monitoring the time period of the connection state due to the transformer, and FIG. 6 is a flowchart illustrating the state when each time period being motioned by the timer is over each preset predetermined time.

### (Step S1) Reset of the time t₁

The inventor sets forth the flowchart of the FIG. 4. Referring to FIG. 4, the starting point of the flowchart is right after the signal related to the control directive is not input into the control circuit 24. First, the time t₁ when the apparatus is not operated is reset as null (0).

### (Step S2) The time t₁ > the predetermined time?

The timer 23 monitors the time t₁ while the signal related to the control directive is not being input into the control circuit 24 from the start time that is null (0) set at the step S1. The control circuit 24 determines whether the time t₁ while the signal related to the control directive is not being input into the control circuit 24, i.e., the time t₁ while the apparatus monitored with the timer 23 is not being operated, becomes longer than the preset predetermined time (time t₁ > predetermined time) or not. If the time t₁ is shorter than preset predetermined time (the case is No in the flowchart in FIG. 4), proceed to the step S3. If the time t₁ is longer than preset predetermined time (the case is Yes in the flowchart in FIG. 4), proceed to A, where the condition A, time t₁ > predetermined time, is met, of the flowchart in FIG. 4.

### (Step S3) input signal?

At the step S2, when the control circuit 24 determines that the time t₁ is shorter than the preset predetermined time, the control circuit 24 determines whether the signal (input signal) related to the control directive is input into the control circuit 24 or not. When the signal related to the control directive is input into the control circuit 24 (the case is Yes in the flowchart in FIG. 4) within the predetermined time from the start time that is null (0) reset at the step S1, the step returns to the step S1 to reset the time t₁, while the apparatus is not being operated, to null (0). When the signal related to the control directive is not input into the control circuit 24 (the case is No in the flowchart in FIG. 4) within the predetermined time from the start time that is null (0) reset at the step S1, the step returns to the step S2 and it is determined that such a condition (time t₁> the predetermined time) is met or not.

### (Step T1) Connection is present?

Next, the inventor sets forth the flowchart of the FIG. 5. First, the transformer 22 determines whether the apparatus per se electrically connects with the power outlet C (connection?) or not. When the apparatus per se is not electrically connecting with the power outlet C (the case is No in the flowchart in FIG. 5), the step returns to the step T1 and the standby loop is maintained until the transformer 22 determines that the apparatus per se electrically connects with the power outlet C. When the apparatus per se electrically connects with the power outlet C (the case is Yes in the flowchart in FIG. 5), the step proceeds to the step T2.

### (Step T2) Reset of the time t₂

The transformer 22 resets the time t₂ under the connection state to null (0) as the starting point that is right after the apparatus per se electrically connects with the power outlet C at the step T1.

### (Step T3) Time t₂ > the predetermined time?

The control circuit 24 determines whether the time t₂ under the connection state due to the transformer 22, monitored by the timer 23, as the starting point (i.e., right after the apparatus per se electrically connects with the power outlet C at the step T1) that is reset to null (0) at the step T2, is longer than the preset predetermined time (i.e., time t₂ > the predetermined time) or not. If the time t₂ is shorter than preset predetermined time (the case is No in the flowchart in FIG. 5), proceed to the step T4. If the time t₂ is longer than preset predetermined time (the case is Yes in the flowchart in FIG. 5), proceed to B, where the condition B (time t₂ > predetermined time), is met, of the flowchart in FIG. 5.

### (Step T4) Connection is present?

At the step T3, when the control circuit 24 determines that the time t₂ is shorter than the preset predetermined time, the transformer 22 determines whether the state, in which the apparatus per se electrically connects with the power outlet C, is maintained (the connection is present?) or not. The point different from the step T1 is that the timer 23 monitors the time t₂ as the starting point (i.e., right after the apparatus per se electrically connects with the power outlet C at the step T1) that is reset to null (0) at the step T2. When the apparatus per se is disconnected from the power outlet C (the case is No in the flowchart in FIG. 5) within the predetermined time from the start time that is null (0) reset at the step T2, the step returns to the step T1 and the standby loop is maintained until the transformer 22 detects that the apparatus per se electrically connects with the power outlet C. When the state, in which the apparatus per se is electrically connecting with the power outlet C, is maintained within the predetermined time from the start time that is null (0) reset at the step T2 (the case is Yes in the flowchart in FIG. 5), the step returns to the step T3 and it is determined that such a condition (time t₂ > the predetermined time) is met or not.

### (Step U1) AΛ B

Next, the inventor sets forth the flowchart of the FIG. 6. When conditions of the time t₁ while the apparatus monitored by the timer 23 is longer than the preset predetermined time and in addition, the time t₂ under the connection state due to the transformer 22 monitored by the timer 23 is longer than the preset predetermined time, i.e., the time t₁ > the predetermined time (A) and the time t₂ > the predetermined time (B) meet the condition (denoted as AΛB of the flowchart in FIG. 6), the step proceeds to the step U2.

### (Step U2) Start charging the accumulator

When the time t₁ > the predetermined time (A) and the time t₂ > the predetermined time (B) meet the condition at the step U1, the control circuit 24 disconnects the accumulator 7 and the high-voltage generation circuit 26 and starts charging the accumulator 7 through the power outlet C.

The portable X-ray apparatus 1 according to the aspect of the present Embodiment comprises the timer 23 that monitors the time period, the connection detector (transformer 22 of the present Embodiment as detection means) that detect the connection between the outside (external) power source (power outlet C of the present Embodiment) and the apparatus per se and the control unit (control circuit 24 of the present Embodiment), that achieves the control set forth below. other than the X-ray voltage generator (generation circuit) that generates voltage to generate the X-ray (high-voltage generation circuit 26 of the present Embodiment) and the accumulator 7. Specifically, the connection between the accumulator 7 and the X-ray voltage generation circuit 26 (means) is cut off when the suspension time period in which the apparatus monitored by the timer 23 is suspending is longer than the first predetermined time period and in addition, when the connection detection means (transformer 22) monitored by the timer 23 detects that the connection time period is over than the second predetermined time period, and charging the accumulator 7 using the outside power source (power outlet C) starts. In other words, the incident, in which the time when the operation of the apparatus is inactive is over the first predetermined time, means that the apparatus is not in use, and the incident, in which the connection time detected by the connection detector is (transformer 22) over the second predetermined time, means that the apparatus per se is being connected with the outside power source (power outlet C) over the second predetermined time. Accordingly, just once the apparatus per se is connected to the outside power source (power outlet C), the charging of the accumulator 7 begins following cutting off the connection between the accumulator from 7 and the X-ray generation circuit (high-voltage generation circuit 26) based on the result given by the timer 23. As a result, the accumulator 7 is automatically charged by just connecting to the outside power source (power outlet C) regardless of the activation-and-suspension of the apparatus. In addition, the accumulator 7 is automatically charged by just connecting with the outside power source (power outlet C, so that a possible chance in which the user forgets to charge the accumulator 7 is effectively minimized.

In addition, according to that the digital imaging such as an FPD is getting popular, some users need to take an image immediately after the operation while the apparatus is being activated and kept in standby in such as the operation room. In such a case, the activated apparatus is continuously connecting with the power outlet and is standing by. During such a standby duration, the apparatus per se (here, i.e., the control circuit 24) determines automatically whether charging is needed or not just by connecting the apparatus per se to the power outlet C and starts charging the accumulator 7. Therefore, the remaining capacity level of the accumulator 7 is maintained to be available by charging the accumulator 7, so that the imaging or the fluoroscopy can be performed with no time-loss. In addition, the lead accumulator applied to the portable X-ray apparatus 1 of the present Embodiment is automatically charged and the accumulator is maintained in the state in which the accumulator is always and almost fully charger, and as a result, the life of the accumulator can be effectively elongated.

In addition, according to the aspect of the present invention, the portable X-ray apparatus 1 comprises a circuit or a component (instrumentation) that consumes less power than the power consumed by the X-ray voltage generation means (high-voltage generation circuit 26). Now, as set forth in the above 'Means for solving the problem', the circuit and instrumentation that consume less power than the power consumed by the X-ray voltage generation means (high-voltage generation circuit 26) is the circuit or the instrumentation does not require a power consumption due to a large electric current in a short period of time, i.e., a high-rate discharge of the accumulator 7 is not required. Such a circuit and a instrumentation include a below connection-disconnection switching circuit (key-switching circuit 25 of the present Embodiment), the above control circuit 24, the charging circuit 21, the FPD, and the personal computer monitor and so forth.

It is preferred that when the apparatus per se is being electrically connected with the outside power source (power outlet C), the power is supplied to such a circuit or instrumentation from the outside power source (power outlet C), and when the apparatus per se is not being electrically connected with the outside power source (power outlet C), the power is supplied thereto from the accumulator 7. Such circuits and instrumentation does not require the high-rate discharge of the accumulator 7, so that when the apparatus per se is being electrically connected with the outside power source (power outlet C), an automatic charging is carried out and even when charging the accumulator 7 and the power supply to such circuit and instrumentation are simultaneously underway, the charging control for the accumulator 7 is correctly achieved without causing the rapid voltage drop in the accumulator 7. On the other hand, when the apparatus per se is not electrically connected to the outside power source (power outlet C), the accumulator 7 supplies power to such circuits and instrumentation. Therefore, regardless of the connection state between the outside power source (power outlet C) and the apparatus per se, the power is supplied to such circuits and instrumentation and as a result, such circuits and instrumentations enable a processing.

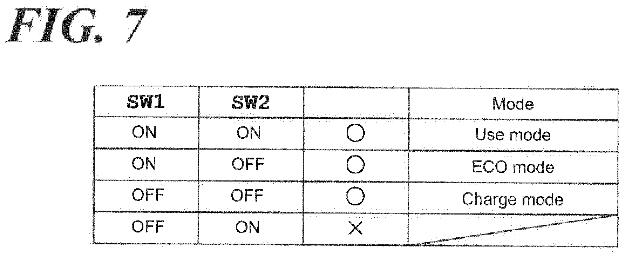

According to the aspect of the present Embodiment as set forth above, when the circuits and instrumentations electrically connect with the outside power source (power outlet C), the power is supplied to the circuits and instrumentations from the outside power source (power outlet C), and when the circuits and instrumentations do not electrically connect with the outside power source (power outlet C) and the power is supplied thereto from the accumulator, but even when the power is not supplied according to the aspect of the present Embodiment, the connection-disconnection switching circuit can be equipped to switch the connection and disconnection between the accumulator 7 and the X-ray voltage generation circuit (high-voltage generation circuit 26). According to the aspect of the present Embodiment, such connection-disconnection switching circuit is an operable circuit through the switching operation from outside, and when the key-switching circuit 25 is adopted as an example, the key-switching circuit 25 functions as illustrated referring to FIG. 7. FIG. 7 is illustrating on-and-off modes of the key-switching circuit.

Three on-and-off modes are available for the key-switching circuit 25 as set forth in 'Objects to be Solved' (refer to 'O' in FIG. 7.) The first mode is a mode by which once the user turns on the first switch (SW1), each target second switch (SW2) between the two electric circuits and instrumentations is turned on to shunt the two electric circuits and instrumentations to connect electrically to one another. Reversely, the second mode is a mode by which once the user turns on the first switch (SW1), the second switch (SW2) between the two of electric circuit and instrumentation is switched to off to open-and-separate the circuit and instrumentation, and such a second mode functions as ECO (energy saving economical) mode to suppress the standby-power (phantom load) due to the connection. Usually, the key-switching circuit is applied to the power switch, so that the key operation, in which the user turns off the first switch (SW1), means that the power switch of the apparatus turns off. Accordingly, once the user turns off the first switch (SW1), the second switches (SW2) between the two targets of electric circuit and instrumentation automatically turn off. Accordingly, once the user turns off the first switch (SW1), the power switch turns off, so that the switching mode, in which the target second switches (SW2) between the two targets of electric circuits and instrumentations turn on, is not possible (refer to 'X' in FIG. 7).

When the connection-disconnection switching circuit as such a key-switching circuit 25, which is externally operable with a switch, is applied to connection-disconnection between the accumulator and 7 the X-ray voltage generation circuit according to the aspect of the present Embodiment, the results are as shown in FIG. 7. With regard to the mode in which the second switch (SW2) between the accumulator 7 and the X-ray voltage generation means (high-voltage generation circuit 26) circuit turns on to electrically connect, when the first switch (SW1) turns on, the accumulator 7 supplies the power for the X-ray voltage generation means (high-voltage generation circuit 26) of the apparatus to be used (refer to 'Use mode' in FIG. 7). With regard to the mode in which the second switch (SW2) between the accumulator 7 and the X-ray voltage generation means (high-voltage generation circuit 26) turns off to disconnect, when the first switch (SW1) turns on, such mode is used as ECO mode while the apparatus is in standby (refer to 'ECO' mode in FIG. 7). With regard to the mode in which the second switch (SW2) between the accumulator 7 and the X-ray voltage generation means (high-voltage generation circuit 26) automatically turns off, when the first switch (SW1) off on, the accumulator 7 is solely charged (refer to 'Charge mode' in FIG. 7).

The connection-disconnection switching circuit as the conventional key-switch circuit, which is externally operable with a switching operation, defers from the control means (e.g., control circuit) and connects with the accumulator, and the power is supplied to the connection-disconnection switching circuit from the accumulator (refer (c) in FIG. 3 prior art). According to such a conventional aspect, for example, when the accumulator is charged under the above ECO mode, the high-current from the accumulator that is connected in series does not flow into the X-ray voltage generation circuit separated from the accumulator but flows into the connection-disconnection switching circuit (key-switching circuit) connected with the accumulator. As set forth above, the connection-disconnection switching circuit (key-switching circuit) is the circuit that does not require a high-efficient discharge performance of the accumulator. As a result, the high-frequency current due to the high-current flows into the connection-disconnection switching circuit (key-switching circuit) and may cause a noise.

Then, referring to FIG. 2, it is preferred that when the apparatus per se electrically connects with the outside power source (power outlet C), the connection-disconnection switching circuit (key-switching circuit 25) connects with the outside power source (power outlet C), so that the power is supplied from the outside power source (power outlet C) to the connection-disconnection switching circuit (key-switching circuit 25), and when the apparatus per se electrically does not connect with the outside power source (power outlet C), the connection-disconnection switching circuit (key-switching circuit 25) connects with the accumulator 7, so that the power is supplied from the accumulator 7 to the connection-disconnection switching circuit (key-switching circuit 25). In such a case, when charging the accumulator 7, the apparatus per se connects with the outside power source (power outlet C), so that even the accumulator 7 is being charged under the above ECO mode, the power is supplied from the outside power source (power outlet C) to the connection-disconnection switching circuit (key-switching circuit 25), so that it is preventable that the high-current from the accumulator 7 flows into the connection-disconnection switching circuit (key-switching circuit 25).

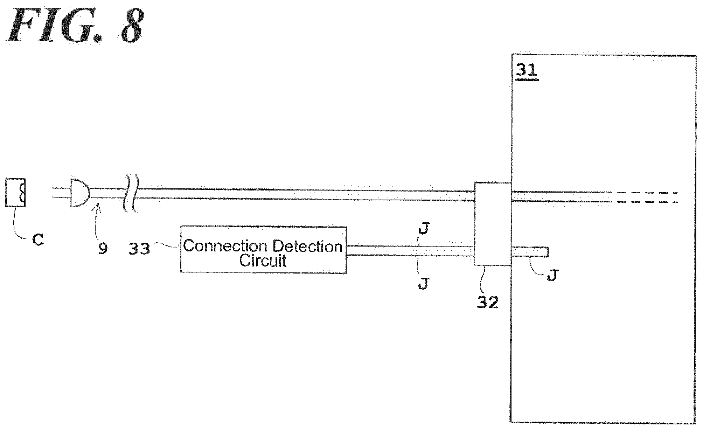

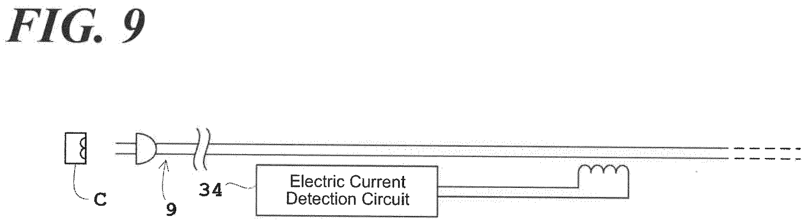

The present invention is not limited to the aspect of the Embodiment set forth above and further another alternative Embodiment can be implemented set forth below.
(1) According to the aspect of the Embodiment set forth above, the inventor sets forth the portable X-ray apparatus used in the visiting imaging in hospital, for the emergency imaging in the patient room and in the operation room, as examples, the X-ray apparatus is not particularly limited thereto as long as the X-ray apparatus has an X-ray voltage generation means (high-voltage generation circuit 26 in the Embodiment) to generate a voltage for generating X-ray. For example, the X-ray apparatus can be a retrofit (fixed) X-ray apparatus. However, the application of the present invention is useful to a portable X-ray apparatus given performing imaging or fluoroscopy using the accumulator without the power wall-outlet in the periphery of the apparatus, in which no outside power source is available.
(2) According to the aspect of the Embodiment set forth above, the outside power source is the power outlet, but the outside power source can have an own power generation function.
(3) According to the aspect of the Embodiment set forth above, the detection means that detects the connection between the outside power source (the power outlet C in the Embodiment) and the apparatus per se is the transformer 22 referring to FIG. 2, but the connection detection means that detects the connection is not always limited to the transformer. For example, referring to FIG. 8, the X-ray apparatus may comprise the substrate circuit 31 having an embedded high-voltage generation circuit (not shown in FIG. 8), a connector 32 insertable into the substrate circuit 31 and a connection detection circuit 33. The substrate circuit 31 electrically connects with the power cable 9 and a jumper wire J via the connector 32, and the jumper wire J electrically connects with the connection detection circuit 33. The plug of the power cable 9 is inserted into the power outlet C and also the connector 32 is inserted into the substrate circuit 31 to connect the apparatus per se and the power outlet C. Accordingly, the jumper wire J also electrically connects with the substrate circuit 31 and as a result, the connection detection circuit 33 detects an electric potential of the jumper wire J, so that the connection between the apparatus per se and the power outlet C is detected.
(4) According to the aspect of the present Embodiment and the above alternative Embodiment 3, the connection between the outside power source (power outlet C in the Embodiment and the alternative Embodiment 3) and the apparatus per se is detected by detecting the electric potential, but also the connection between the outside power source (power outlet C) and the apparatus per se can be detected by detecting the current flowing in the power cable. For example, referring to FIG. 9, the X-ray apparatus may comprise the electric current detection circuit 34. The electric current detection circuit 34 detects the current flowing in the power cable 9. The plug of the power cable 9 is inserted into the power outlet C to electrically connect the apparatus per se and the power outlet C. In such a way, the electric current detection circuit 34 detects the current flowing in the power cable 9, so that the connection between the apparatus per se and the power outlet C can be detected.
(5) According to the aspect of the Embodiment set forth above, the control means is the control circuit comprising circuits having the CPU and so forth, but such control means can be a programmable device (e.g., field programmable gate array, FPGA) of which the hardware circuit (e.g., logic circuit) used inside can be changed correspondingly to the program data.
(6) According to the aspect of the Embodiment set forth above, whereas the control circuit 24 (referring to FIG. 2) comprises the timer 23 (referring to FIG. 2), the control means represented by such as the control circuit 24 does not have to always embed the timer. The control means (circuit) and the timer can separately equipped.
(7) According to the aspect of the Embodiment set forth above, when the apparatus per se electrically connects with the outside power source (power outlet C in the Embodiment), the power is supplied from the outside power source (power outlet C) to the circuits and instrumentations, which consume less power than the power consumed by the X-ray voltage generation means (high-voltage generation circuit 26 in the Embodiment), and on the other hand, when the apparatus per se does not electrically connect with the outside power source (power outlet C), the power is supplied from the accumulator 7, but the present invention is not limited to such power supply methods. Even when the apparatus per se electrically connects with the outside power source (power outlet C), the accumulator may supply power to such circuits and instrumentation from the accumulator while charging the accumulator. In addition, the power supply method, in which the power is supplied from the accumulator or the outside power source (power outlet C) to the circuits and instrumentations, can be switched depending on the remaining capacity of the accumulator. For example, when not charging the accumulator and the remaining capacity of the accumulator is a little, the power is supplied from the outside power source (power outlet C) to the circuits and instrumentations, and on the other hand, when charging the accumulator or the remaining capacity of the accumulator is enough, it is switched so that the power is supplied from the accumulator to the circuits and instrumentations.
(8) According to the aspect of the Embodiment set forth above, the connection-disconnection switching circuit is an operable circuit (key-switching circuit 25 in the Embodiment) using the switch from the outside, but not limited to an operable circuit using the switch from the outside. In addition, an operable circuit (key-switching circuit in the Embodiment) using the switch from the outside is not limited to a key-switching circuit that is operated by the switch in synchronism with the key operation. For example, such a circuit may be operable in synchronism with pressing down the switch.
(9) When automatically charging the accumulator, charging may be conducted when the remaining capacity is less than the constant amount by checking the remaining capacity of the accumulator based on the detection of voltage variation of the accumulator, which is carried out by that a tiny amount of current is flowed prior to charging with high-current to prevent over charging.

### Industrial Applicability

As set forth above, the present invention is suitable for a variety of portable X-ray imaging apparatuses.

### Reference of signs

- 1: Portable X-ray apparatus
- 7: Accumulator
- 22: Transformer
- 23: Timer
- 24: Control circuit
- 25: Key-switching circuit
- 26: High-voltage generation circuit
- SW1: First switch
- SW2: Second switch
- C: Power outlet

Having described at least one of the preferred embodiments of the present invention with reference to the accompanying drawings, it will be apparent to those skills that the invention is not limited to those precise embodiments, and that various modifications and variations can be made in the presently disclosed system without departing from the scope or spirit of the invention. Thus, it is intended that the present disclosure cover modifications and variations of this disclosure provided they come within the scope of the appended claims and their equivalents.

## Claims

1. An X-ray apparatus, having an X-ray voltage generation circuit that generates a voltage for X-ray generation, comprising:
an accumulator;
a timer that monitors a time period;
a connection detection circuit that detects a connection between an outside power source and said X-ray apparatus; and
a control circuit; wherein:
said control circuit disconnects said connection between said accumulator and said X-ray voltage generation circuit when a suspension time period, in which said apparatus monitored by said timer is not being operated, is longer than a first preset predetermined time period and said connection detection circuit detects that a connection time period is longer than a second preset predetermined time period, and starts charging said accumulator using an outside power source.

2. The X-ray apparatus, according to Claim 1, wherein:
at least one component selected from a group consisting of a circuit and an instrumentation each of which consumes a lesser power than a power consumed by said X-ray power generation circuit; and
wherein said outside power source supplies at least said one component from said group consisting of said circuit and said instrumentation with a power when said X-ray apparatus electrically connects with said outside power source; and said accumulator supplies said one component from said group consisting of said circuit and said instrumentation with power when said X-ray apparatus does not electrically connect with said outside power source.

3. The X-ray apparatus, according to Claim 2, wherein:
said at least one component that consumes the less power than the power consumed by said X-ray power generation circuit is a connection-disconnection switching circuit that respectively connects and disconnects a connection between said accumulator and said X-ray voltage generation circuit;
said outside power source supplies said connection-disconnection switching circuit with a power when said X-ray apparatus electrically connects with said outside power source; and
said accumulator supplies said connection-disconnection switching circuit with power when said X-ray apparatus does not electrically connect with said outside power source.

4. The X-ray apparatus, according to Claim 1 wherein:
a connection-disconnection switching circuit that connects-disconnects said accumulator and said X-ray voltage generation circuit.

5. The X-ray apparatus, according to Claim 3, wherein:
said connection-disconnection switching circuit is operable by switching from said outside power source.

6. The X-ray apparatus, according to Claim 4, wherein:
said connection-disconnection switching circuit is operable by switching from said outside power source.

7. The X-ray apparatus, according to Claim 5 or Claim 6, wherein:
said outside power source supplies said connection-disconnection switching circuit with a power when said X-ray apparatus electrically connects with said outside power source; and
and said accumulator supplies said connection-disconnection switching circuit with power when said X-ray apparatus does not electrically connect with said outside power source.
